Europäisches Patentamt

European Patent Office

Office européen des brevets

(19)

(11) Publication number: **0 191 349 B1**

(12) **EUROPEAN PATENT SPECIFICATION**

(45) Date of publication of patent specification: **01.04.92**

(51) Int. Cl.5: **C07K 7/00**, A61K 37/00, G01N 33/68

(21) Application number: **86101063.5**

(22) Date of filing: **27.01.86**

The file contains technical information submitted after the application was filed and not included in this specification

(54) **A peptide compound and antiserum produced by using this compound.**

(30) Priority: **15.02.85 SE 8500712**

(43) Date of publication of application:
**20.08.86 Bulletin 86/34**

(45) Publication of the grant of the patent:
**01.04.92 Bulletin 92/14**

(84) Designated Contracting States:
**AT BE CH DE FR GB IT LI LU NL**

(56) References cited:

**CHEMICAL ABSTRACTS, vol. 107, 1987, abstract no. 37578y, Columbus, Ohio, US; M. NAKAZATO et al.: "Biochemical nature of familial amyloidotic polyneuropathy and a new diagnostic method by radioimmunoassay", & AMYLOIDOSIS, (PROC. INT. SYMP. AMYLOIDOSIS:DIS. COMPLEX) 4th 1984 (PUB. 1986) 407-14**

(73) Proprietor: **Gambro Lundia AB
Box 10101
S-220 10 Lund(SE)**

(72) Inventor: **Grubb, Anders
Gilleskroken 5
S-222 47 Lund(SE)**
Inventor: **Hansson, Heléne
Simrishamnsgatan 3
S-214 23 Malmö(SE)**
Inventor: **Ljunggren, Lennart
Jullovsvägen 6
S-223 67 Lund(SE)**

(74) Representative: **Boberg, Nils Gunnar Erik
Gambro AB Patent Department Box 10101
S-220 10 Lund(SE)**

CHEMICAL ABSTRACTS, vol. 87, 1977, page 431, abstract no. 20426t, Columbus, Ohio, US; IGNACZAK et al.: "Immunochemical studies on the nature of the serum component (SAA) related to secondary amyloidosis", & J. LAB. CLIN. MED. 1977, 89(5), 1092-104

BRITISH MEDICAL JOURNAL, vol. 288, 4th February 1984, pages 360-361; C.P.J. MAURY et al.: "Measurement of serum amyloid A protein concentrations as test renal allograft rejection in patients with initially non-functioning grafts"

BRITISH MEDICAL JOURNAL, vol. 288, 17th March 1984, pages 859-860; M.B. PEPYS: "Measurement of serum amyloid A protein concentrations as test of renal allograft rejection"

PROC. NATL. ACAD. SCI. USA, vol. 78, no. 6, June 1981, pages 3824-3828; T.P. HOPP et al.: "Prediction of protein antigenic determinants from amino acid sequences"

BIOCHEMISTRY, vol. 21, no. 14, 6th July 1982, pages 3298-3303, American Chemical Society; D.C. PARMELEE et al.: "Amino acid sequence of amyloid-related apoprotein (apoSAA1) from human high-density lipoprotein"

## Description

### TECHNICAL FIELD

Peptide compound and antisera, produced by the use of such compound, will be described.

The invention relates in particular to peptide compounds for the production of antisera which are specific for amyloid A protein and/or serum amyloid A protein (hereafter called AA and SAA respectively) and which consequently constitute valuable means for the detection and the determination of the concentration of the said proteins.

### BACKGROUND ART

In many chronic illnesses, such as rheumatoid arthritis, tuberculosis, hepatitis and infectious and tumerous affections there is a strong increase in the blood content of SAA. Through the splitting off of the 28 last amino-acid residues from SAA in such affection AA is formed in organs of these patients to such an extent that they cease to function. The organs which are hit earliest are the kidneys and the heart. This illness manifestation is called amyloidosis and, if not treated, is lethal.

To make it possible to detect amyloidosis at an early stage and thus allow treatment of this illness manifestation through increased action against the basic affection, a method is required for the detection of AA in tissue specimens and/or the determination of the SAA content in blood samples.

A known method which is based on the production of antisera which are specific for AA and/or SAA is described in the British Medical Journal, vol. 288, 4th February 1984. As far as we know this known method has not become generally accessible since it has proved particularly difficult to produce antisera specific for AA and/or SAA. Moreover, the method has been subject of certain criticism (see British Medical Journal, vol. 288, 17th March 1984, pp. 859-60.

It is an object of the invention, therefore, to overcome the difficulty of the known technique and thereby lay the foundation of a well-functioning method of the type described above.

The object is achieved in accordance with the invention with the help of the peptide compunds and antisera which are defined in the following claims and which will be described in greater detail hereunder.

Reference is also being made to Biochemistry 21, 1982, pages 3298-3303, which discloses the sequence of the natural occuring apoSAA (a precursor of the tissue amyloid protein AA).

Reference is furthermore being made to Chemical Abstract 107, 1987, page 308; 107: 37578, Amyloidosis (Proc. Int. Symp. Amyloidosis:

Diss. Complex) 4th 1984, pages 407-414, and Chemical Abstract 87, 1977, page 431; 87: 20426t, J. Lab. Clin. Med. 89(5), 1977, pages 1092-1104.

### DISCLOSURE OF INVENTION

A peptide compound of the type which is specified in the preamble to claim 1 is thus produced in accordance with the invention. The characteristic feature of the peptide compound is that the number of amino-acid residues is at least 6 and in that the said sequence is built up of the following amino-acid residues:
Ser-Asp-Ala-Arg-Glu-Asn-Ile-Gln-Arg.

The expression "hydrophilicity value" is accepted in the field and is used in accordance with the invention exactly in the manner as described in Proc. Natl. Acad. Sci. USA, vol.78, no. 6, pp. 3824-3828, June 1981. In this literature reference is also found to a table of such values for particular amino-acid residues.

The reason why the number of amino-acid residues in the peptide compound in accordance with the invention should be greater than 6, is explained more fully, for example, in the Nature, vol. 299, 14th October 1982, p. 596 for synthetic peptide compounds in general.

The peptide compound in accordance with the invention can be prepared synthetically by using in principle conventional methods in the field. Even though it doesn't in general involve any major difficulties to synthesize a peptide compound with a predetermined sequence of amino-acids, it is none the less surprising that a synthetic peptide compound in accordance with the invention can be used for the production of a specific antiserum for AA and/or SAA, bearing in mind that it is particularly difficult when the natural proteins are to serve as an antigen, on the one hand because it is difficult to isolate them wholly free from any contaminating substances, on the other hand because they are difficultly soluble or insoluble in ordinary buffers. Moreover, the peptide conceivable for immunization can be chosen in many differnt manners, since no completely reliable method exists for choosing the "right" peptide.

A peptide compound prepared by synthesis in accordance with the invention has been found to function well for precisely the preparation of specific antisera for AA and/or SAA. Said peptide compound is a nonapeptide with the following amino-acid sequence:
Ser-Asp-Ala-Arg-Glu-Asn-Ile-Gln-Arg
where, in known manner, the codes used have the following meanings

    Ser =    serine
    Asp =    aspartic acid
    Ala =    alanine

Arg = arganine
Glu = glutamic acid
Asn = asparagine
Ile = isoleucine
Gln = glutamine

In accordance with the invention an antiserum is produced also for the detection of AA in a tissue specimen. The antiserum is characterized in that it is procuded by using a petide coumpund of the type described above, i e the nonapeptide of the following amino-acid sequence:

Ser-Asp-Ala-Arg-Glu-Asn-Ile-Gln-Arg

Furthermore, an antiserum for the determination of the concentration of SAA in blood is produced in accordance with the invention which is characterized in that it is produced by using a peptide compound of the type described above, i e a nonapeptide with the following amino-acid sequence:

Ser-Asp-Ala-Arg-Glu-Asn-Ile-Gln-Arg

The nonapeptide defined above, coupled to a high molecular carrier was injected in accordance with conventional technique into five rabbits, two times at three weeks' interval. All the rabbits produced antisera which reacted specifically with AA in body tissues as well as with SAA in blood in immunohistochemical techniques and in immunoblood techniques of known type. The production of antiserum continued without loss of titre during six weeks without renewed injections of nonapeptide carrier complex and therefor can be regarded as stable.

INDUSTRIAL APPLICABILITY

Peptide compounds in accordance with the invention are specially useful for the production of specific antisera for AA and/or SAA.

Antisera prepared by using peptide compounds in accordance with the invention are therefore specially useful for the detection of AA in tissue specimens and/or determination of SAA concentration in blood.

Claims

1. A peptide compound containing amino-acid residues which occur naturally in amyloid A protein and/or serum amyloid A protein, characterized in that the number of amino-acid residues is at least 6 and in that the said sequence is built up of the following amino-acid residues: Ser-Asp-Ala-Arg-Glu-Asn-Ile-Gln-Arg.

2. Use of the peptide compound in accordance with claim 1 for the preparation of antisera.

3. An antiserum for the detection of amyloid A protein in a tissue specimen, characterized in that it is produced using a peptide compound in accordance with claim 1.

4. An antiserum for the determination of the concentration of serum amyloid A protein in a blood sample, characterized in that it is produced using a peptide compound in accordance with claim 1.

Revendications

1. Composé peptidique contenant des résidus d'acide aminés qui apparaissent naturellement dans la protéine amyloïde A et/ou dans la protéine sérum-amyloïde A, caractérisé en ce que le nombre de résidus d'acides aminés est d'au moins 6 et en ce que ladite séquence est constituée des résidus d'acides aminés suivants : Ser-Asp-Ala-Arg-Glu-Asn-Ile-Gln-Arg.

2. Utilisation du composé peptidique selon la revendication 1 pour la préparation d'antisérums.

3. Antisérum pour la détection de la protéine amyloïde A dans un échantillon de tissu, caractérisé en ce qu'on le prépare en utilisant un composé peptidique selon la revendication 1.

4. Antisérum pour la détermination de la concentration de la protéine sérum-amyloïde A dans un échantillon de sang, caractérisé en ce qu'on le prépare en utilisant un composé peptidique selon la revendication 1.

Patentansprüche

1. Peptidverbindung, die Aminosäurereste enthält, welche natürlich in Amyloid A-Protein und/oder Serumamyloid A-Protein vorkommen, **dadurch gekennzeichnet**, daß die Anzahl der Aminosäurereste wenigstens 6 beträgt und daß diese Sequenz aus den folgenden Aminosäureresten aufgebaut ist: Ser-Asp-Ala-Arg-Glu-Asn-Ile-Gln-Arg.

2. Verwendung der Peptidverbindung nach Anspruch für die Herstellung von Antiseren.

3. Antiserum für die Ermittlung von Amyloid A-Protein in einer Gewebeprobe, **dadurch gekennzeichnet,** daß es unter Verwendung einer Peptidverbindung nach Anspruch 1 hergestellt ist.

4. Antiserum zur Bestimmung der Konzentration von Serumamyloid A-Protein in einer Blutprobe, **dadurch gekennzeichnet,** daß es unter Verwendung einer Peptidverbindung nach Anspruch 1 hergestellt ist.